Europäisches Patentamt

European Patent Office

Office européen des brevets

(11) Publication number : **0 485 209 A1**

## (12) EUROPEAN PATENT APPLICATION

(21) Application number : **91310297.6**

(22) Date of filing : **07.11.91**

(51) Int. Cl.⁵ : **C12N 15/40,** C12Q 1/70,
C07K 15/00, G01N 33/576

(30) Priority : **08.11.90 JP 304405/90**

(43) Date of publication of application :
**13.05.92 Bulletin 92/20**

(84) Designated Contracting States :
**BE CH DE DK FR GB IT LI NL**

(71) Applicant : **IMMUNO JAPAN INC.**
**4-28-14-701, Ogikubo Suginami-Ku**
**Tokyo 167 (JP)**

(72) Inventor : **Okamoto, Hiroaki**
**3132-24, Yakushi Ji, Minami Kawachi Machi**
**Kawachi-Gun, Tochigi-Ken (JP)**
Inventor : **Nakamura, Tetsuo**
**4-28-14-701 Ogikubo**
**Suginami-ku, Tokyo 167 (JP)**

(74) Representative : **Arthur, Bryan Edward**
**4 Dyers Buildings Holborn**
**London, EC1N 2JT (GB)**

(54) **Non A, non B hepatitis virus related antigen, antibody detection systems, polynucleotides and polypeptides.**

(57) Non-A, non-B hepatitis (NANB hepatitis) virus RNA and its corresponding polypeptide, related antigen, antibody, and detection systems for detecting NANB hepatitis antigen or antibodies.

EP 0 485 209 A1

Jouve, 18, rue Saint-Denis, 75001 PARIS

## Background of the Invention

The present invention relates to non-A, non-B hepatitis (hereinafter called NANB hepatitis) virus RNA and its corresponding polypeptide, related antigen, antibody, and detection systems for detecting NANB hepatitis antigen or antibodies.

Viral hepatitis (of which the DNA and the RNA of the causative viruses have been elucidated and their diagnosis, and even prevention in some cases, have been established) is caused by hepatitis A, hepatitis B, hepatitis D and hepatitis E. In spite of great efforts by scientists the world over, however, the causative virus of NANB hepatitis (falling in none of the above categories and mainly caused by blood born infection) has not been isolated.

In 1988, Chiron Corp. claimed that they had succeeded in cloning the RNA virus genome, which they termed hepatitis C virus (hereinafter called HCV) as the causative agent of NANB hepatitis and reported on its nucleotide sequence. HCV antibody detection sytems based on the sequence are now being introduced for screening of blood for transfusion and for diagnosis of patients. Detection systems for the HCV antibody have proven their partial association with NANB hepatitis; however, they capture only about 70% of carriers and chronic hepatitis patients, or they fail to detect the antibody in acute phase infection, thus leaving problems yet to be solved even after development of the HCV antibody by Chiron Corp.

On the other hand, detection methods for hepatitis B virus have been established. More than 95% of post-transfusion cases of hepatitis in Japan are caused by NANB hepatitis (with annual estimated cases of 280,000). The course of NANB hepatitis is troublesome and most patients are considered to become carriers, then to develop chronic hepatitis. In addition, those patients with chronic hepatitis develop liver cirrhosis, then hepatocellular carcinoma at fairly high rates over 10 to 20 years. It is therefore very imperative to isolate the virus itself and to develop effective diagnostic reagents enabling earlier diagnosis.

The presence of a number of NANB hepatitis cases which can not be diagnosed by Chiron's HCV antibody detection kits demands development of NANB hepatitis diagnostic kits of more specificity. To develop such kits, it becomes an absolutely important task to analyze the causative virus of NANB hepatitis at its genetic (and corresponding amino acid) level.

## Summary of the Invention

An object of this invention is to provide the nucleotide sequence coding for the structural protein of NANB hepatitis virus and, with such information, to analyze amino acids of the protein to locate and provide polypeptides useful as antigens for establishment of detection systems for NANB virus, its related antigens and antibodies.

A further object of the present invention is to locate polypeptides effective as antigens by isolating NANB hepatitis virus RNA from human and chimpanzee virus carriers, cloning the cDNA covering the whole structural gene of the virus to determine its nucleotide sequence, and studying the amino acid sequence of the cDNA. As a result, a specific region of the structural gene of the virus coding for the core protein was determined.

## Brief Description of the Drawings

Figure 1a, 1b, and 1c represent restriction maps of the nucleotide sequences of 5′ termini of NANB hepatitis virus strains HC-J1, HC-J4 and HC-J5 respectively. Solid lines show nucleotide sequences determined by clones from libraries of bacteriophage lambda gt11, and broken lines show nucleotide sequences determined by clones obtained by PCR.

Figure 2 is a plot of hydrophilicity of amino acids of the structural protein of NANB hepatitis virus.

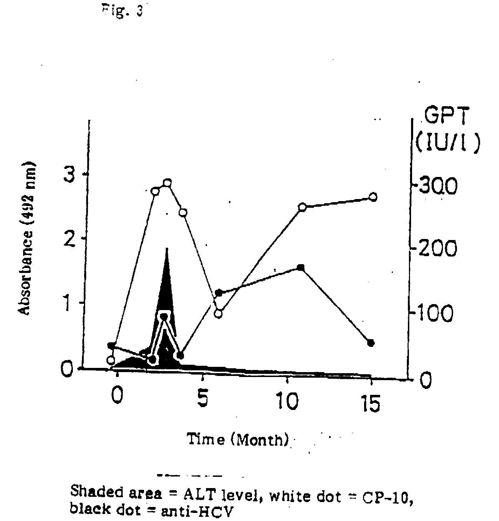

Figure 3 is a graph showing the time-course of ALT, anti-CP9, and anti-HCV levels in a post-transfusion NANB hepatitis patient.

## Detailed Description of the Invention

Abbreviations used in the present invention are as follows: For RNA, A, G, C and U stand for Adenine, Guanine, Cytosine and Uracil respectively. For DNA, A, G and C indicate the same bases as in RNA and T stands for Thymine. For polypeptides, A, R, N, D, C, E, Q, G, H, I, L , K, M, F, P, S, T, W, Y, and V show the respective amino acids Alanine, Arginine, Asparagine, Aspartic Acid, Cysteine, Glutamic Acid, Glutamine, Glycine, Histidine, Isoleucine, Leucine, Lysine, Methionine, Phenylalanino, Proline, Serine, Threonine, Tryptophan, Tyrosine and Valine.

In the method described below, NANB hepatitis virus RNA under this invention was obtained and its nucleotide sequence was determined.

Four samples (HC-J1, HC-J2, HC-J4 and HC-J5) were obtained from human and chimpanzee plasma. HC-J1 and HC-J5 were obtained from Japanese blood donors who tested positive for HCV antibody. HC-J2 was obtained from a chronic NANB patient with its infectivity confirmed by chimpanzee challenge test. HC-J4 was obtained from the chimpanzee subjected to the challenge test but was negative for Chiron's HCV antibody previously mentioned.

RNA was isolated from each of the four plasma samples and the homology of the nucleotide sequences were compared with each other. Respective nucleotide sequences of 5′ terminus of RNA isolated from HC-J1 and HC-J4 (which had the least homology to each other) and that from HC-J5 were determined (as described in the Examples).

As shown in Example 1, strains HC-J1, HC-J4 and HC-J5 had a 5′ noncoding region consisting of at least 341 nucleotide followed by a region coding for the structural protein (nt342-nt1880 for strains HC-J1 and HC-J4, and nt342-nt1886 for strain HC-J5). Structural gene region of both HC-J1 and HC-J4 strains were made up of 1539 nucleotides and are identical in the number of nucleotides. The number of nucleotides of the structural gene region of the HC-J5 strain was 1545.

When compared with the sequence of HCV deiclosed in European Patent Application No. 88310922.5 (by Chiron Corp.), the homology of the sequences under this invention in comparison to theirs was 92.7s% for strain HC-J1, 70.2% for strain HC-J4 and only 57.4% for strain HC-J5 in the limited sequence of 191 nucleotides (nt1690-nt1880); the only part which was comparable between the sequences of strains HC-J1, HC-J4, and HC-J56 and those disclosed in the European Patent Application. Nucleotides under this invention were presumed to be followed by the 7310 nucleotide sequence of HCV at nt1690. The region coding for a polypeptide, including the structural proteins, constitutes a long open reading frame and consisted of 8658 nucleotides, and the sequence described in the above-described European Patent Application was considered to be the region coding only for the non-structural protein.

Based on the nucleotide sequences of strains HC-J1, HC-J4 and HC-J5, sequences of 513 amino acids (515 for strain HC-J5) coded for by the structural gene region of NANB hepatitis virus, and shown in the Examples, were determined.

When the amino acid sequences of strains HC-J1 and HC-J4 were compared, there was higher amino acid sequence homology in the upstream region than in the downstream region, and the upstream region was hydrophilic (containing more basic amino acids like arginine). Basic amino acids tend to be found in the core protein of various viruses providing affinity with nucleic acids; therefore, this upstream region was considered to code for the core protein while the dowstream region (where frequent displacement of amino acids as many as eight glycosylation sites were found) was considered to be coding for the surface protein.

Homology of amino acid sequences of strains HC-J1 and HC-J5 was 92.1% for amino acids 1-180, 63.0% for amino acids 181-515, and 73.2% throughout the region, and that of strains HC-J4 and HC-J5 was 92.8% for amino acids 1-180, 57.9%, for amino acids 81-515, and 70.1% throughout the region; in both cases there was higher amino acid sequence homology in the upstream than in the downstream region.

Thus, the region coding for the structural protein (which plays an important role in antigenicity of NANB hepatitis virus) was confirmed and its nucleotide sequence was clarified in this invention. And by so doing, the amino acid sequence constituting the structural protein was elucidated to provide decisively important material for searching epitopes having antigenicity.

The above points were not clarified in the above-mentioned European Patent Application by Chiron.

As candidates for peptides having antigenicity, peptides CP-9, CP-10 and CP-5 were automatically chosen based on hydrophilicity scores. Although amino acid sequences for CP-9, CP-10 and CP-5 (as described later) were chosen based on the nucleotide sequence of strain HC-J1, they can be those of strain HC-J4 (disclosed in this invention) or amino acid sequences with amino acid displacement(s) without loss of antigenicity. All of them arm effective and covered under this invention.

As described later, peptides CP-10, CP-9 and CP-5 were synthesized and NANB hepatitis virus diagnosis systems using such peptides were confirmed to be effective for highly specific detection of NANB hepatitis virus infection.

These peptides can not only be chemically synthesized but can also be produced by genetic engineering using cells already known to public.

Applicants also produced antibodies against peptides CP-10, CP-9 and CP-5, and confirmed their usefulness in detection of NANB hepatitis viral antigen.

Peptide CP-10 has the following amino acid sequence:

P K P Q R K T K R N T N R R P Q D V K.

Peptide CP-9 has the following amino acid sequence:

R R G P R L G V R A T R K T S E R S Q P R

G R R Q P I P K V R R P E G R.

Peptide CP-5 has the following amino acid sequence:

R G S R P S W G P T D P R R R S R N L G.

Peptides CP-10, CP-9 and CP-5 (described above, or with partial displacement(s)), or detection systems using antibodies against them, are useful as diagnostic reagents specific to NANB hepatitis, and are effective for screening of NANB hepatitis virus in blood for transfusion or blood derivatives, and hence have high practical value. They are all covered by this invention. Related peptides for CP-9 having its 30th amino acid displaced by A, its 23rd and 30th amino acids displaced by W and A respectively, or its 30th, 33rd, 34th and 36th amino acids displaced by D, S, T and K respectively, have all been confirmed effective. As regards CP-5, a peptide partially related at its 10th, 14th and 19th amino acids by N, H and V respectively was confirmed equally effective.

Including, but not limited to such polypeptides, all peptides having partial displacement(s) in their amino acid sequences (yet retaining antigenicity) are covered by this invention.

The above described peptides, and antibodies against them, can be used as materials for vaccine or passive immunization.

Polynucleotides CN-10, CN-9 and CN-5 (which correspond to the polypeptides described above) have the following sequences:

(CN-10)

```
                                         C C C A
A A C C T C A A A ?      A A A A A C C A A A
C G T A A C A C C A      A C C G T C G C C C
A C A G G A C G T C      A A G;
```

(CN-9)

```
C G C A G G G G C C C T A G A T T G G G T G
T G C G C G C G A C      G A G G A A G A C T
T C C G A G C G G T      C G C A A C C T C G
A G G T A G A C G T      C A G C C T A T C C
C C A A G G T G C G      T C G G C C C G A G
G G C A G G;
```

(CN-5)

C G T G G C     T C T C G G C C T A

G T T G G G G C C C     C A C G G A C C C C

C G G C G T A G G T     C G C G C A A T T T

G G G T.

Each of the polynucleotides under this invention is capable of producing the corresponding polypeptide in host cells by conventional recombinant techniques.

Examples

Examples of this invention are shown below; however, this invention shall in no way be limited to those examples.

Example 1

5′ terminal nucleotide sequence and amino acid sequence of NANB hepatitis virus genome were determined in the following way:

(1) Isolation of RNA

RNA of the sample (HC-J1) from plasma of a Japanese blood donor (who tested positive for HCV antibody by Ortho HCV Ab ELISA, Ortho Diagnostic System, Tokyo), and the sample (HC-J4) from the chimpanzee challenged with NANB hepatitis for infectivity (and negative for HCV antibody), were isolated in the following method.

1.8 ml of the plasma samples was added with 1 ml of Tris chloride buffer (10 mM, pH 8.0) and centrifuged at $68 \times 10^3$ rpm for 1 hour. Its precipitate was suspended in Tris chloride buffer (50 mM, Ph 8.0; containing 200 mM NaCl, 10 mM EDTA, 2% (w/v) sodium dodecyl sulfate (SDS) and 1 mg/ml proteinase K) and was incubated at 60°C for 1 hour. Then the nucleic acids were extracted by phenol/chloroform and precipitated by ethanol to obtain RNAs.

(2) cDNA Synthesis

After heating the RNA isolated from HC-J1 plasma at 70°C for 1 minute, this was used as a template; 10 units of reverse transcriptase (cDNA Synthesis System Plus, Amersham Japan) and pmol of oligonucleotide primer (20 mer) were added and incubated at 42°C for 1.5 hours to obtain cDNA. Primer #8 (5′-GAT GCT TGC GGA AGC AAT CA-3′) was prepared by referring to the basic sequence shown in European Patent Application No. 88310922.5, which is relied on and incorporated herein by reference.

(3) cDNA Was Amplified by the following Polymerase Chain Reaction (PCR)

cDNA was amplified for 35 cycles according to Saiki's method (Science (1988) 239: 487-491) using Gene Amp DNA Amplifier Reagent (Perkin-Elmer.Cetus) on a DNA Thermal Cycler (Perkin-Elmer.Cetus).

(4) Determination of Nucleotide Sequence by Assembling cDNA Clones

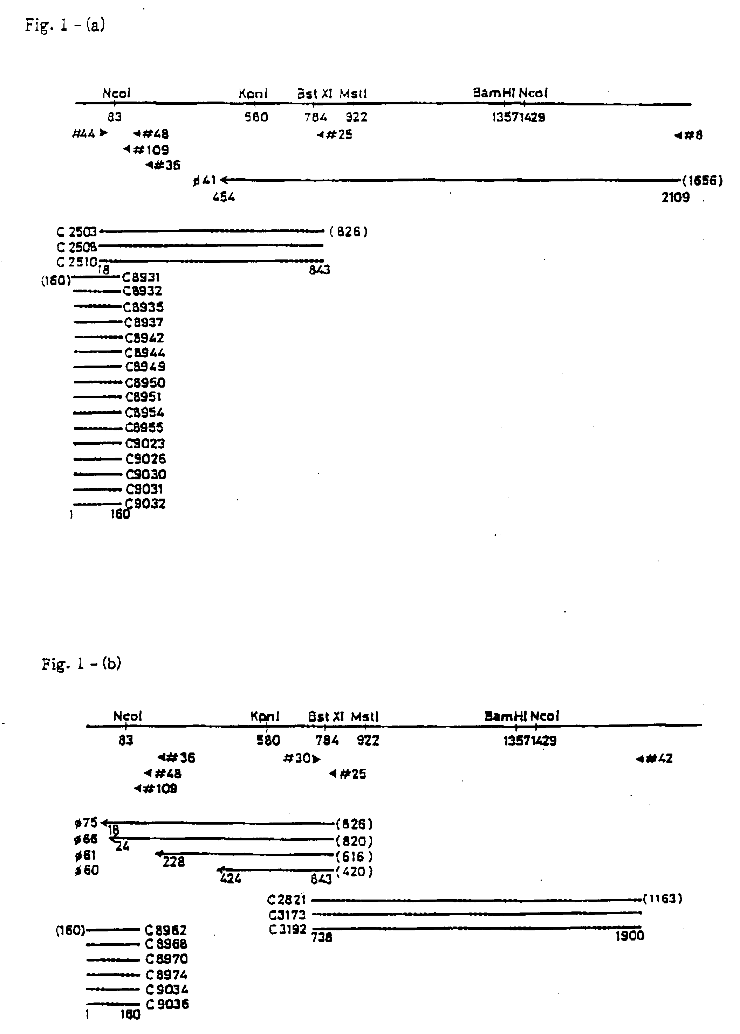

As shown in Fig. 1, nucleotide sequences of 5′ termini of the genomes of strains HC-J1, HC-J4 and HC-J5 were determined by combined analysis of clones obtained from the cDNA library constructed in bacteriophage lambda gt10 and clones obtained by amplification of HCV specific cDNA by PCR. Fig. 1 shown 5′ terminus of NANB hepatitis virus genome together with cleavage site by restriction endonuclease and sequence of primers used. In Fig. 1, solid lines are nucleotide sequences determined by clones from bacteriophage lambda gt10 library while dotted lines show sequences determined by clones obtained by PCR.

A 1656 nucletide sequence of HC-J1 (spanning nt454-2109) was determined by clone ø41 which was obtained by inserting the cDNA synthesized with the primer #8 into lambda gt10 phage vector (Amersham).

Primer #25 (5'-TCC CTG TTG CAT AGT T CAC G-3') of nt824-843 wazs synthesized based on the ø41 sequence, and four clones (ø60, ø61, ø66 and ø75) were obtained to cover the upstream sequence nt18-843.

To determine the extreme upstream of the 5' terminus, single-stranded cDNA was sybthesized using antisense primer #36 (5'-A ACA CTA CTC GGC TAG CAG T -3') of nt246-265, and then it was added with dATP tail at its 3' terminus by terminal deoxynucleotidyl transferase, then amplified by one-sided PCR in two stages. That is, in the first stage, oligo dT primer (20-mer) and antisense primer #48 (5'-GTT GAT CCA AGA AAG G ACC C-3') of nt188-207 were used to amplify the dA-tailed cDNA by PCR for 35 cycles; and in the second stage, using the product of the first-stage PCR as a template, oligo dT primer (20-mer) and antisense primer #109 (21-mer; 5'-ACC GGA TC CGC AGA CCA CTA T-3') of nt140-160 were added to initiate PCR for 30 cycles. The product of PCR was subcloned to M13 phage vector. Six independent clones (C8962, C8968, C8970, C8974, C9034 and C9036), were obtained (each considered having complete length of 5' terminus), and the nucleotide sequence of nt1-17 of the respective clones was determined.

The upstream sequence of strain HC-J1, in the region of nt18-843, was determined by clones (C2503, C2508 and C2510 which were obtained by PCR amplification by the primers #44 (5'-GGCGACACTCCACCATAGAT-3') and #25 (5'-TCCCTGTTGCATAGTTCACG-3'). Nucleotide sequence of 5' terminus further upstream, nt1-37, was determined by 16 clones, C8931, C8932, C8935, C8937, C8942, C8944, C8949, C8950, C8951, C8954, C8955, C9023, C9026, C9030, C9031 and C9032 obtained by the same method an for the HC-J4 strain.

The dowstream sequence from nt738 to 1900 of strain HC-J4 (having 1163 nucleotides) was determined by three clones of C2821, C3173 and C3192 which were obtained by PCR amplification by primers #30 (5'-CTCATGGGGTACATTCCGCT-3') and #42 (5'-TCGGTCGTCCCCACCACAAC-3').

Isolation of RNA from HC-J5, and determination of its sequence, was conducted in the same manner as described above.

Sequences in the range of nt21-1886 of the RNA were determined by respective clones obtained by amplification by PCR utilizing each pair of primers shown below:


## nt24-265

#32 (5'- A C T C C A C C A T A G A T C A C T C C -3')

#36 (5'- A A C A C T A C T C G G C T A G C A G T -3')

Clones: C3534, C3536, C3537.


## nt63-508

#33 (5'- T T C A C G C A G A A A G C G T C T A G -3')

#51 (5'- G A C C G C T C C G A A G T C T T C C T -3')

Clones: C4658, C4659, C4660.


## nt467-843

#23 (5'- T A G A T T G G G T G T G C G C G C G A -3')

#25 (5'- T C C C T G T T G C A T A G T T C A C G -3')

Clones: C2451, C2453, C2454.

nt732-934

#50 (5'- G C C G A T C T C A T G G G G T A C A T -3')

#63 (5'- C T G G T G T T C T T A A C C T G G A C -3')

Clones: C5592, C5593, C5594.


nt867-1354

#55 (5'- A T T T T C T T G C T G G C C C T G C T -3')

#54 (5'- A T C G C G T A C G C C A G G A T C A T -3')

Clones: C5164, C5303, C5331.


nt1240-1880

#38 (5'- T G C A A T T G T T C T A T C T A C C C -3')

#41 (5'- C A G G G C T T G G G G T G A A G C A A -3')

Clones: C3722, C3748, C3753.


nt1842-1976

#4  (5'- A G T G T G T G T G G T C C G G T A T A -3')

#5  (5'- C G G T G G C C T G G T A T T G T T A A -3')

Clones: C3952, C3969, C3990.


Three cDNA clones (C8995, C8997, C8998) wore obtained in the same way as applied to strains HC-J4 and HC-J1 to determine the nucleotide sequence of nt1-43 for pinning down 5' terminal site.

From the analysis described above, full nucleotide sequences of 5' termini of genomes of strains HC-J1, HC-J4, and HC-J5 were determined, as shown below.

The nucleotide sequence of the genome of strain HC-J1 is first shown below, then those of strains HC-J4 and HC-J5 (with part of the nucleotide sequences underlined to show the difference from HC-J1). nt1-341 is 5' noncoding region and nt342-1880 (nt342-1886 for strain HC-J5) is region coding for the structural protein supposedly starting with initiation codon ATG. The nucleotide sequence shown in the aforementioned European Patent Application started only with the 1690th nucleotide, and missed the upstream sequence which is originally revealed in this invention.

Nucleotide sequence of the genome of strain HC-J1 (N1880-1):

```
      G C C A G C C          C C C T G A T G G G
    G G C G A C A C T C      C A C C A T G A A T
    C A C T C C C C T G      T G A G G A A C T A
    C T G T C T T C A C      G C A G A A A G C G
    T C T A G C C A T G      G C G T T A G T A T
    G A G T G T C G T G      C A G C C T C C A G 117
    G A C C C C C C C T      C C C G G G A G A G
    C C A T A G T G G T      C T G C G G A A C C
    G G T G A G T A C A      C C G G A A T T G C
    C A G G A C G A C C      G G G T C C T T T C
    T T G G A T A A A C      C C G C T C A A T G 217
    C C T G G A G A T T      T G G G C G C G C C
    C C C G C A A G A C      T G C T A G C C G A
    G T A G T G T T G G      G T C G C G A A A G
    G C C T T G T G G T      A C T G C C T G A T
    A G G G T G C T T G      C G A G T G C C C C 317
    G G G A G G T C T C      G T A G A C C G T G
    C A C C A T G A G C      A C G A T T C C C A
    A A C C T C A A A G      A A A A A C C A A A
    C G T A A C A C C A      A C C G T C G C C C
    A C A G G A C G T C      A A G T T C C C G G 417
    G T G G C G G T C A      G A T C G T T G G T
    G G A G T T T A C T      T G T T G C C G C G
    C A G G G G C C C T      A G A T T G G G T G
```

```
TGCGCGCGAC        GAGGAAGACT
TCCGAGCGGT        CGCAACCTCG 517
AGGTAGACGT        CAGCCTATCC
CCAAGGTGCG        TCGGCCCGAG
GGCAGGACCT        GGGCTCAGCC
CGGGTACCCT        TGGCCCCTCT
ATGGCAATGA        GGGCTGCGGG 617
TGGGCGGGAT        GGCTCCTGTC
TCCCCGTGGC        TCTCGGCCTA
GTTGGGGCCC        CACGGACCCC
CGGCGTAGGT        CGCGCAATTT
GGGTAAGGTC        ATCGATACCC 717
TCACGTGCGG        CTTCGCCGAC
CTCATGGGGT        ACATACCGCT
CGTCGGCGCC        CCTCTTGGAG
GCGCTGCCAG        GGCCCTGGCG
CATGGCGTCC        GGGTTCTGGA 817
AGACGGCGTG        AACTATGCAA
CAGGAACCT         TCCTGGTTGC
TCTTTCTCTA        TCTTCCTTCT
GGCCCTGCTC        TCTTGCCTGA
CTGTGCCCGC        TTCAGCCTAC 917
CAAGTGCGCA        ACTCCACAGG
GCTTTATCAT        GTCACCAATG
ATTGCCCTAA        CTCGAGTATT
GTGTACGAGG        CGCACGATGC
```

```
C A T C C T G C A T          A C T C C G G G G T 1017

G T G T C C C T T G          C G T T C G C G A G

G G C A A C G T C T          C G A G G T G T T G

G G T G G C G A T G          A C C C C C A C G G

T A G C C A C C A G          G G A C G G C A A A

C T C C C C G C G A          C G C A G C T T C G 1117

A C G T C A C A T C          G A T C T G C T T G

T C G G A G C G C            C A C C C T C T G T

T C G G C C C T C T          A C G T G G G G A

T C T G T G C G G G          T C C G T C T T C C

T T A T T G G T C A          A C T G T T T A C C 1217

T T C T C T C C C A          G G C G C C A C T G

G A C A A C G C A A          G G C T G C A A T T

G T T C T A T C T A          C C C C G G C C A T

A T A A C G G G T C          A T C G C A T G G C

A T G G A T A T G            A T G A T G A A C T 1317

G G T C C C C T A C          G G C G G C G T T G

G T A A T G G C T C          A G C T G C T C C G

G A T C C C A C A A          G C C A T C T T G G

A T A T G A T C G C          T G G T G C T C A C

T G G G A G T C C            T G G C G G G C A T 1417

A G C G T A T T T C          T C C A T G G T G G

G G A A C T G G C            G A A G G T C C T G

G T A G T G C T G T          T G C T G T T T G C

C G G C G T C G A C          G C G G A A A C C A

T C G T C T C C G G          G G G A C A A G C C 1517
```

```
G C C C G C G C C A        T G T C T G G A C T

T G T T A G T C T C        T T C A C A C C A G

G C G C T A A G C A        G A A C A T C C A G

C T G A T C A A C A        C C A A C G G C A G

T T G G C A C A T C        A A T A G C A C G G 1617

C C T T G A A C T G        C A A T G A A A G C

C T T A A C A C C G        G C T G G T T A G C

A G G G C T T A T C        T A T C A A C A C A

A A T T C A A C T C        T T C G G G C T G T

C C C G A G A G G T        T G G C C A G C T G 1717

C C G A C G C C T T        A C C G A T T T T G

A C C A G G G C T G        G G G C C C T A T C

A G T C A T G C C A        A C G G A A G C G G

C C C C G A C C A A        C G C C C T A T T

G T T G G C A C / /        / / / / T A C C C C 1817

C C A A A A C C T T        G C G G T A T C G T

G C C C G C A A A G        A G C G T A T G T G

G C C C G G T A T A        T T G C T T C A C T

C C C A G C C C C  (1886-6=1880).
```

Nucleotide sequence of the genome of strain HC-J4 (N1880-2):

```
        G C C A G C C        C C C G A T T G G G

      G G C G A C A C T C        C A C C A T A G A T

      C A C T C C C C T G        T G A G G A A C T A

      C T G T C T T C A C        G C A G A A A G C G

      T C T A G C C A T G        G C G T T A G T A T
```

GAGTGTCGTG　　CAGCCTCCAG 117

GACCCCCCCT　　CCCGGGAGAG

CCATAGTGGT　　CTGCGGAACC

GGTGAGTACA　　CCGGAATTGC

CAGGACGACC　　GGGTCCTTTC

TTGGATCAAC　　CCGCTCAATG 217

CCTGGAGATT　　TGGGCG<u>T</u>GCC

CCCGC<u>G</u>AGAC　　TGCTAGCCGA

GTAGTGTTGG　　GTCGCGAAAG

GCCTTGTGGT　　ACTGCCTGAT

AGGGTGCTTG　　CGAGTGCCCC 317

GGGAGGTCTC　　GTAGACCGTG

CACCATGAGC　　ACGA<u>A</u>TCC<u>T</u>A

AACCTCAAAG　　AAAAACCAAA

CGTAACACCA　　ACCG<u>C</u>CGCCC

ACAGGACGTC　　AAGTTCCCGG 417

G<u>C</u>GG<u>T</u>GGTCA　　GATCGTTGGT

GGAGTTTAC<u>C</u>　　TGTTGCCGCG

CAGGGGCCC<u>C</u>　　AG<u>G</u>TTGGGTG

TGCGCGCGAC　　<u>T</u>AGGAAGACT

TCCGAGCGGT　　CGCAACCTCG 517

<u>T</u>GG<u>AT</u>GGCG<u>A</u>　　CA<u>A</u>CCTATCC

CCAAGG<u>CT</u>CG　　<u>C</u>CG<u>A</u>CCCGAG

GGCAGG<u>G</u>CCT　　GGGCTCAGCC

CGGGTACCCT　　TGGCCCCTCT

ATGGCAATGA　　GGGCTT<u>GG</u>GG 617

```
T G G G C A G G A T        G ?         ? G T C

A C C C C G C G G C        T C C G G C C T A

G T T G G G G C C C        C A C G G A C C C C

C G G C G T A G G T        C G C G T A A C T T

G C T A A G G T C          A T C G A T A C C C 717

T T A C A T G T G G        C T T C G C C G A T

C T C A T G G C G T        A T A T T C C G C T

C G T C G G C G C C        C C C C T A G G G G

G C G C T G C C A G        G G C C T T G G C A

C A C G G T G T C C        G G G T T C T G G A 817

G G A C G G C G T G        A A C T A T G C A A

C A G G A A C T T          G C C C G G T T G C

T C T T T C T C T A        T C T T C C T C T T

G G C T T T G C T G        T C C T G T T T G A

C C A T C C C A G C        T T C C G C T T A T 917

G A A G T G C G C A        A C G T G T C C G G

G A T A T A C C A T        G T C A C G A A C G

A C T G C T C C A A        C T C A A G C A T T

G T G T A T G A G G        C A G C G G A C A T

G A T C A T G C A T        A C T C C C G G G T 1017

G C G T G C C C T G        C G T T C G G G A G

G A C A A C A G C T        C C C G T T G C T G

G G T A G C G C T C        A C T C C C A C G C

T C G C G G C C A G        G A A T G C C A G C

G T C C C C A C T A        C G A C A A T A C G 1117

A C G C C A C G T C        G A C T T G C T C G
```

13

```
T T G G G G C G G C          T G C T T T C T G C

T C C G C T A T G T          A C G T G G G G A

T C T C T G C G G A          T C T G T T T T C C

T C G T C T C C C A          G C T G T T C A C C  1217

T T C T C G C C T C          G C C G G C A T G A

G A C A G T G C A G          G A C T G C A A C T

G C T C A A T C T A          T C C C G G C C A T

T T A T C A G G T C          A C C G C A T G G C

T T G G G A T A T G          A T G A T G A A C T  1317

G G T C A C C T A C          A A C A G C C C T A

G T G G T G T C G C          A G T T G C T C C G

G A T C C C A C A A          G C T G T C G T G G

A C A T G G T G G C          G G G G G C C C A C

T G G G G A G T C C          T G G C G G G C C T  1417

T G C C T A C T A T          T C C A T G G T A G

G G A A C T G G G C          T A A G G T C C T G

A T T G T G G C G C          T A C T C T T C G C

C G G C G T T G A C          G G G G A G A C C T

A C A C G T C G G G          G G G G G C G G C C  1517

A G C C A C A C C A          C C T C C A C G C T

C G C G T C C C T C          T T C T C A C C T G

G G G C G T C T C A          G A G A A T C C A C

C T T G T G A A T A          C C A A C G G C A G

C T G G C A C A T C          A A C A G G A C T G

C C C T A A A C T G          C A A T G A C T C C

C T C C A C A C T G          G G T T C C T T G C
```

```
C G C G C T G T T C        T A C A C A C A C A
G G T T C A A C T C        G T C C G G G T G C
C C G G A G C G C A        T G G C C A G C T G 1717
C C G C C C A T T          G A C T G G T T C G
C C C A G G G A T G        G G G C C C C A T C
A C C T A T A C T G        A G C C T G A C A G
C C C G G A T C A G        A G G C C T T A T T
G C T G G C A T / /        / / / / T A C G C G 1817
C C T C G A C C G T        G T G G T A T C G T
A C C C G C G T C G        C A G G T G T G T G
G T C C A G T G T A        T T G C T T C A C C
C C A A G C C C T (1886-6=1880)
```

Nucleotide sequence of the genome of strain HC-J5 (N1886):

```
A C C C G C C             C C C T A A T A G G
G G C G A C A C T C       C G C C A T G A A C
C A C T C C C C T G       T G A G G A A C T A
C T G T C T T C A C       G C A G A A A G C G
T C T A G C C A T G       G C G T T A G T A T
G A G T G T C G T A       C A G C C T C C A G 117
G C C C C C C C C T       C C C G G G A G A G
C C A T A G T G G T       C T G C G G A A C C
G G T G A G T A C A       C C G G A A T T G C
C G G G A A G A C T       G G G T C C T T T C
T T G G A T A A A C       C C A C T C T A T G 217
C C C G G C C A T T       T G G G C G T G C C
```

```
C C C G C A A G A C          T G C T A G C C G A

G T A G C G T T G G          G T T G C G A A A G

G C C T T G T G G T          A C T G C C T G A T

A G G G T G C T T G          C G A G T G C C C C 317

G G G A G G T C T C          G T A G A C C G T G

C A C C A T G A G C          A C A A A T C C C A

A A C C T C A A A G          A A A A A C C A A A

A G A A A C A C T A          A C C G T C G C C C

A C A A G A C G T T          A A G T T T C C G G 417

G C G G C G G C C A          G A T C G T T G G C

G G A G T A T A C T          T G T T G C C G C G

C A G G G G C C C C          A G G T T G G G T G

T G C G C G C G A C          A A G G A A G A C T

T C G G A G C G G T          C C C A G C C A C G 517

T G G G A G G C G C          C A G C C C A T C C

C C A A A G A T C G          G C G C T C C A C T

G G C A A G T C C T          G G G G A A A G C C

A G G A T A T C C C          T G G C C C C T A T

A T G G G A A T G A          G G G G C T C G G C 617

T G G G C A G G A T          G G C T C C T G T C

C C C C C G A G G T          T C C C G T C C C T

C T T G G G G C C C          C A A T G A C C C C

C G G C A T A G G T          C G C G C A A T G T

G G G T A A G G T C          A T C G A T A C C C 717

T A A C G T G C G G          C T T T G C C G A C

C T C A T G G G G T          A C A T C C C C G T
```

CGTGGGCGCC

GCGTCGCCAG

CACGGCGTGA

GGACGGGGTT

CAGGGAACTT

TCCTTTTCTA

GGCGCTACTG

CCGTCCCGGT

CAGGTTAAGA

CAGCTATATG

ACTGTTCCAA

ACCTGGCAGC

GGTTCTCCAC

GCGTCCCGTG

GGGAATATGT

GATACCGGTC

TGGCTGTGCG

GCCCTCACGC

GACGCACATC

TGGTGTCCGC

TCCGCTCTCT

CCTCTGCGGT

TCGCGGCCCA

GTCTCGCCGC

GTTTGTGCAG

GCTCCATCTA

CCGCTTGGTG

AGCTCTCGCA

GAGTCCTGGA 817

AACTATGCAA

ACCTGGTTGC

TTTTCTTGCT

TCCTGCATCA

CTCTGCTGTC 917

ACACCAGTAA

GTGACTAACG

TGACAGCATT

TCCAGGGCGC

GTCCCCGGGT 1017

CGAGAAAGTG

CACGGTGCTG

TCACCGAACG

GCAGCCCGGC

AGGGTCTGCG 1117

GACATGGTTG

TACGCTCTGC

ACGTGGGGGA

GGGGTGATGC

GATGTTCATC 1217

AGCACCACTG

GAATGCAATT

CCCTGGTGCC

ATCACTGGAC        ACCGTATGGC

ATGGGACATG        ATGATGAACT 1317

GGTCACCCAC        GGCCACCATG

ATCCTGGCGT        ACGCGATGCG

CGTCCCCGAG        GTTATCATAG

ACATCATTAG        CGGGGCTCAC

TGGGGCGTCA        TGTTCGGCCT 1417

AGCCTACTTC        TCTATGCAGG

GAGCGTGGGC        GAAGGTCGTT

GTCATCCTTC        TGCTGGCCGC

TGGAGTGGAT        GCGAACACCC

GCACCGTTGC        GGGTTCTGCT 1517

GCGGCAACCA        CCAGGGGCTT

CACCAGCATG        TTCTCCTCTG

GCTCGAAGCA        GAACCTTCAG

CTCATTAACA        CCAACGGTAG

CTGGCACATC        AACCGCACTG 1617

CCCTGAATTG        CAATGACTCC

TTGAACACCG        GCTTTATCGC

GTCCCTGTTC        TACGTCAACC

GCTTCAATTC        GTCAGGATGT

CCCCATCGCC        TGTCCGTCTG 1717

CCGCAGCATC        GAGGCTTTCC

GGATAGGGTG        GGGCACCTTG

CAATACGAGG        ATAATGTCAC

CAATCCAGAA        GATATGAGAC

```
C A T A T T G C T G      G C A C T A C C C A 1817

C C A A A A C C G T      G T G G C A T A G T

C C C C G C G A G G      T C T G T G T G C G

G C C C A G T G T A      C T G T T T C A C C

C C C A G C C C A  1886.
```

5. <u>Determination of Amino Acid Sequences.</u>

According to the nucleotide sequences of the genome of strains HC-J1, HC-J4 and HC-J5, determination was made of the sequences of 513 amino acids (515 amino acids for strain HC-J5) encoded by 1539 nucleotides (excluding 341 nucleotides of 5′ noncoding region) of the structural gene region nt342-1880 (1545 nucleotides) of the structural gene region nt342-1886 for strain HC-J5).

The amino acid sequences of the respective genomes are shown below in order of strain HC-J1, HC-J4 and HC-J5. The sequence of the genome of strain HC-J4 which is underlined shows the partial displacements.


**Amino acid sequence of the genome of strain HC-J1**

**(P513-1):**

```
M S T I P K P Q R K      T K R N T N R R P Q

D V K P P G G G Q I      V G G V Y L L P R R

G P R L G V R A T R      K T S E R S Q P R G

R R Q P I P K V R R      P E G R T W A Q P G

Y P W P L Y G N E G      C G W A G W L L S P 100

R G S R P S W G P T      D P R R R S R N L G
```

```
K V I D T L T C G F        A D L M G Y I P L V
G A P L G G A A R A        L A H G V R V L E D
G V N Y A T G N L P        G C S F S I F L L A
L L S C L T V P A S        A Y Q V R N S T G L 200
Y H V T N D C P N S        S I V Y E A H D A I
L H T P G C V P C V        R E G N V S R C W V
A M T P T V A T R D        G K L P A T Q L R R
H I D L L V G S A T        L C S A L Y V G D L
C G S V F L I G Q L        F T F S P R R H W T 300
T Q G C N C S I Y P        G H I T G H R M A W
D M M M N W S P T A        A L V M A Q L L R I
P Q A I L D M I A G        A H W G V L A G I A
Y F S M V G N W A K        V L V V L L L F A G
V D A E T I V S G G        Q A A R A M S G L V 400
S L F T P G A K Q N        I Q L I N T N G S W
H I N S T A L N C N        E S L N T G W L A G
L I Y Q H K F N S S        G C P E R L A S C R
R L T D P D Q G W G        P I S H A N G S G P
D Q R P Y C W H Y P        P K P C G I V P A K 500
S V C G P V Y C F T        P S P   513.
```

Amino acid sequence of the genome of strain HC-J4 (P513-2):

```
M S T N P K P Q R K        T K R N T N R R P Q
D V K F P G G G Q I        V G G V Y L L P R R
G P R L G V R A T R        K T S E R S Q P R G
W R Q P I P K A R R        P E G R A W A Q P G
```

```
Y P W P L Y G N E G      L G W A G W L L S P 100
R G S R P S W G P T      D P R R R S R N L G
K V I D T L T C G F      A D L M G Y I P L V
G A P L G G A A R A      L A H G V R V L E D
G V N Y A T G N L P      G C S F S I F L L A
L L S C L T I P A S      A Y E V R N V S G I 200
Y H V T N D C S N S      S I V Y E A A D M I
M H T P G C V P C V      R E D N S S R C W V
A L T P T L A A R N      A S V P T T I R R
H V D L L V G A A A      F C S A M Y V G D L
C G S V F L V S Q L      F T F S P R R H E T 300
V Q D C N C S I Y P      G H L S G H R M A W
D M M M N W S P T T      A L V V S Q L L R I
P Q A V V D M V A G      A H W G V L A G L A
Y Y S M V G N W A K      V L I V A L L F A G
V D G E T Y T S G G      A A S H T T S T L A 400
S L F S P G A S Q R      I Q L V N T N G S W
H I N R T A L N C N      D S L H T G F L A A
L F Y T H R F N S S      G C P E R M A S C R
P I D W F A Q G W G      P I T Y T E P D S P
D Q R P Y C W H Y A      P R P C G I V P A S 500
Q S C G P V Y C F T      P S P   513.
```

Amino acid sequence of the genome of strain HC-J5 (P515):

```
M S T N P K P Q R K          T K R N T N R R P Q
D V K P P G G G Q I          V G G V Y L L P R R
G P R L G V R A T R          K T S E R S Q P R G
R R Q P I P K D R R          S T G K S W G K P G
Y P W P L Y G N E G          L G W A G W L L S P  100
R G S R P S W G P N          D P R H R S R N V G
K V I D T L T C G F          A D L M G Y I P V V
G A P L G G V A R A          L A H G V R V L E D
G V N Y A T G N L P          G C S F S I F L L A
L L S C I T V P V S          A V Q V K N T S N S  200
Y M V T N D C S N D          S I T W Q L Q G A V
L H V P G C V P C E          K V G N M S R C W I
P V S P N V A V R Q          P G A L T Q G L R T
H I D M V V V S A T          L C S A L Y V G D L
C G G V M L A A Q M          F I V S P Q H H W F  300
V Q E C N C S I Y P          G A I T G H R M A W
D M M M N W S P T A          T M I L A Y A M R V
P E V I I D I I S G          A H W G V M F G L A
Y P S M Q G A W A K          V V V I L L L A A G
V D A N T R T V A G          S A A A T T R G F T  400
S M F S S G S K Q N          L Q L I N T N G S W
H I N R T A L N C N          D S L N T G F I A S
L F Y V N R F N S S          G C P H R L S V C R
S I E A F R I G W G          T L Q Y E D N V T N


P E D M R P Y C W H          Y P P K P C G I V P  500
A R S V C G P V Y C          F T P S P.
```

6. Determination of the Virus Core Regions.

There was 83% homology between the two sequences (totaling 513 amino acids) constituting the structural protein of strains HC-J1 and HC-J4. Homology between the sequences of the two genomes was 97.2% for upstream amino acids 1-180 and was reduced to 75.4% for dowstream amino acids 181-513.

Comparison between the amino acid sequences encoded by the genomes of strains HC-J1 and HC-J5 showed homology of 92.2% for amino acids 1-180, 63.0% for amino acids 181-515, and 73.2% as a whole; and comparison between the amino acid sequences encoded by the genomes of strains HC-J4 and Hc-J5 showed homology of 92.8% for amino acids 1-180, 57.9% for amino acids 181-515, and 70.1% as a whole; both showing higher homology in the upstream region than in the downstream region.

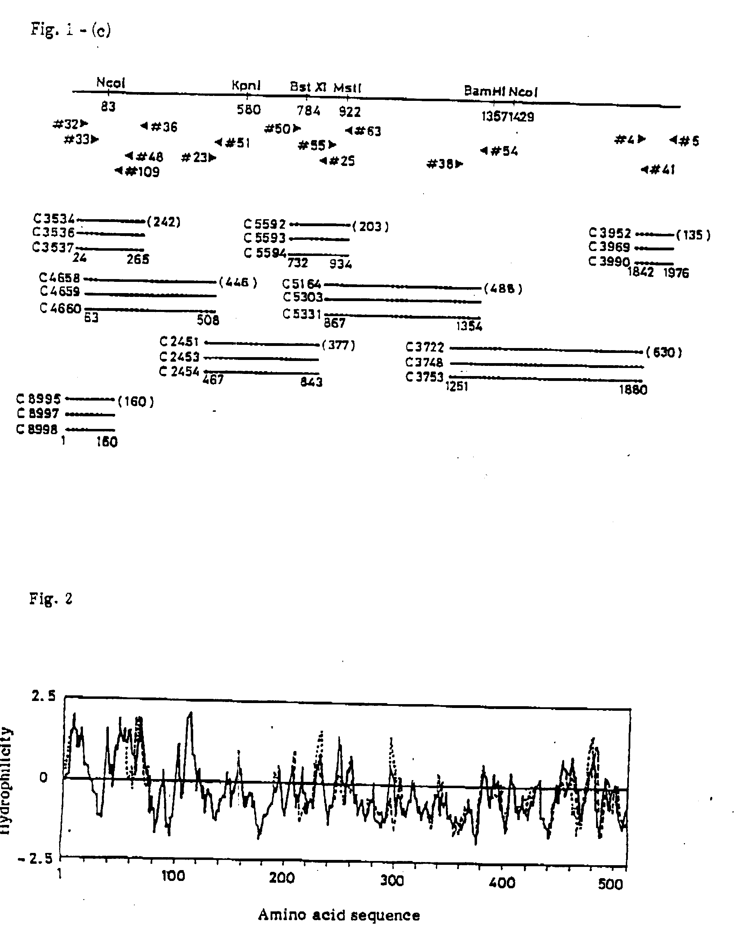

As regards hydrophilicity of amino acids 1-513, three highly hydrophilic parts were noted in amino acids 1-120 (Figure 2). These parts were rich in basic amino acids such as arginine (28/120 = 23%) and the upstream was considered to code for the core protein of NANB hepatitis virus.

From the study of hydrophilicity scores, Applicants had presumed the following sequence of 36 amino acids (CP-9) from 39 to 74 to form an epitope of the core protein:

R R G P R L G V R A T R K T S E R S Q P R G R R Q P I

P K V R R P E G R.

The nucleotide sequence of the gene coding for this amino acid sequence is as follows (CP-9, corresponding sequence is also shown in nt456-563 of the nucleotide sequence):

C G C A G G G G C C C T A G A T T G G G T G

T G C G C G C G A C     G A G G A A G A C T

T C C G A G C G G T     C G C A A C C T C G

A G G T A G A C G T     C A G C C T A T C C

C C A A G G T G C G     T C G G C C C G A G

G G C A G G.

By means of genetic engineering technique, CN-9 can produce CP-9 in host cells such as *Escherichia coli*.

Determination of the epitope of the core protein of NANB hepatitis virus opened access to chemical synthesis of the peptide, manufacturing of the peptide by genetic engineering techniques, synthesis of the polynucleotides, manufacturing of the antibody, manufacturing of NANB hepatitis diagnostic reagents, and development of products such as NANB hepatitis vaccines.

Example 2.

Using the polypeptide CP-9 (39-74) having the amino acid sequence show in Example 1, the inventors established the following detection system for the antibody against NANB hepatitis virus.

1. Manufacture of Peptide

According to the well-known method described by Merrifield, the inventors synthesized peptide CP-9 having the amino acid sequence of R - R - G - P - R - L - G - V - R - A - T - R - K - T - S - E - R - S - Q - P - R - G - R - R - Q - P - I - P - K - V - R - P - E - G - R.

Similarly, peptide CP-10 having the amino acid sequence of P K P Q R K T K R N T N R R P Q D V K and peptide CP-5 having the amino acid sequence of R G S R P S W G P T D P R R R S R N L G were synthesized.

The nucleotide sequence of the gene coding for the amino acid sequence of CP-5 is as follows (CN-5, this corresponds to nt642-701 of the polynucleotide sequence):

```
        C  G  T  G  G  C          T  C  T  C  G  G  C  C  T  A

     G  T  T  G  G  G  G  C  C     C  A  C  G  G  A  C  C  C  C

     C  G  G  C  G  T  A  G  G  T  C  G  C  G  C  A  A  T  T  T

     G  G  G  T.
```

As already described, the peptide CP-10 having the sequence of P K P Q R K T K R N T N R R P Q D V K was synthesized and the nucleotide sequence of the gene coding for the amino acid sequence of CP-10 is as follows (CN-10, this corresponds to nt254-410 of the polynucleotide sequence):

```
                                              C  C  C  A
     A  A  C  C  T  C  A  A  A  G     A  A  A  A  A  C  C  A  A  A

     C  G  T  A  A  C  A  C  C  A     A  C  C  G  T  C  G  C  C  C

     A  C  A  G  G  A  C  G  T  C     A  A  G.
```

CN-5 and CN-10 are capable of expressing CP-5 and CP-10 in host cells by means of genetic engineering techniques.

2. Detection System for Antibody Against NANB Hepatitis Virus

The detection system was developed using polyvinyl microtiter plates and the sandwich method.

In this example, 50µl of 5µg/ml concentration of the peptide was dispensed in each well and incubated overnight at room temperature for consolidation. The microplate wells were washed five times with physiological saline containing 0.05% Tween 20. For overcoating, 100 µl of NaCl buffer containing 30% (v/v) of calf serum and 0.05% Tween 20 (CS buffer) was dispensed in each well and discarded after incubation for 30 minutes at room temperature.

For determination of samples, in the primary reaction, 50µl of the CS buffer containing 30% calf serum and 10 µl of a sample was dispensed in each microplate well and incubated on a microplate vibrator for one hour at room temperature.

After completion of the reaction, microplate wells were washed five times in the same way as previously described.

In the secondary reaction, as labeled antibody 1 ng of horseradish peroxidase labeled anti-human IgG mouse monoclonal antibodies (Fab' fragment: 22G, Institute of Immunology Co., Ltd., Tokyo, Japan) dissolved in 50 µl of calf serum was dispensed in each microplate well, and was incubated on a microplate vibrator for one hour at room temperature. Wells were washed five times in the same way. After addition of hydrogen peroxide (as substrate) and 50 µl of O-phenylendiamine solution (as color developer) in each well, and after incubation for 30 minutes at room temperature, 50 µl of 4M sulphuric acid was dispensed in each well to stop further color development anf for reading absorbance at 492 nm.

The cut-off level of this assay system was set by measuring a number of donor samples with normal ALT value of 34 Karmen unit or below and which tested negative for anti-HCV and its mean absorbance was plus 0.25 (0.05 + 0.25 = 0.30)

Experimental Example.

Five peptides (P19-36, P27-36, P19-27, P10-27 and P10-18)of different length in sequence relating to CP-9 (P39-74, 36 mer), considered to be constituting the epitope of the core region, were synthesized and their inhibition capability was tested.

In the test, 30 µl of the peptide (adjusted to 100 µl/mg; same volume for each sample) was mixed and measured after incubation for two hours at 37°C according to the method described in Example 1. Results are shown in Table 1.

Table 1.   Epitope mapping on CP-9 by peptide-inhibition.

| Inhibition % | < anti-CP-9 (+) > | | |
|---|---|---|---|
| | 1 | 2 | 3 |
| CP9 | 100 | 97 | 96 |
| P19-36 | 80 | 91 | 49 |
| P27-36 | 63 | 82 | 36 |
| P19-27 | 51 | 71 | 20 |
| P10-27 | 39 | 52 | 40 |
| P10-18 | 31 | 33 | 21 |

From the inhibition rate of samples 1 and 2 in the test of positive anti-CP9 samples, it was confirmed that the CP-9 was antigenic throughout its sequence.

Experimental Example.

The detection system for antibody against NANB hepatitis virus developed and described in Example 2 was compared with the conventional anti-HCV detection kit by testing samples from patients with post-transfusion NANB hepatitis and patients with liner diseases. The results proved that the detection system under this invention was much superior to the conventional detection kit both in specificity and sensitivity for the acute phase of the disease.

1- Post-transfusion NANB Hepatitis.

This example concerns a patient who received seven units of blood (via transfusion over two times) and was infected with NANB hepatitis (which manifested itself about two months after transfusion). In this example, blood samples were retrospectively tested by the anti-HCV test kit and the kit developed under this invention; as shown in Table 2, one donor blood sample turned out to have an Absorbance over 2.000 and was positive.

Table 2.  Detection of anti-CP9 and anti-HCV in donor bloods.

| Donors | Antibody | |
| --- | --- | --- |
| | anti-CP9 | anti-HCV |
| No. 1 | 0.082 (-) | 0.018 (-) |
| No. 2 | >2.000 (+) | 0.006 (-) |
| No. 3 | 0.027 (-) | 0.007 (-) |
| No. 4 | 0.086 (-) | 0.063 (-) |
| No. 5 | 0.064 (-) | 0.025 (-) |
| No. 6 | 0.026 (-) | 0.005 (-) |
| No. 7 | 0.038 (-) | 0.009 (-) |

As to the recipient, the antibody detection system under this invention showed absorbance in excess of 2 (coincidental to the abrupt rice in ALT levels of the patient two months after transfusion, while it was not until three months after the transfusion that the conventional test kit detected anti HCV). The antibody detection system under the invention thus proved its significant advantage over the conventional anti-HCV teat kit both in specificity for the donor blood sample test and in earlier detection of the antibody in a patient sample.

2. Detection of Anti-CP9 in Sample from Patients with Liver Diseases.

Frequency of anti-CP9 in patients with various liver diseases are shown in Table 3.

Table 3.  Detection by PCR of viral RNA in samples from

patients with NANB hepatitis related liver diseases

positive for anti-CP9 but negative for anti-HCV.

| Sample | Diagnosis* | Age & Sex** | Absorbance at 492 nm | | HCV RNA (PCR) |
|---|---|---|---|---|---|
| | | | anti-CP9 | anti-HCV | |
| 1 | C H | 46M | >2.000 | 0.030 | + |
| 2 | C H | 45M | >2.000 | 0.076 | + |
| 3 | C H | 47M | >2.000 | 0.031 | + |
| 4 | C H | 60F | >2.000 | 0.163 | + |
| 5 | C H | 64F | >2.000 | 0.133 | + |
| 6 | L C | 47M | >2.000 | 0.253 | - |
| 7 | L C | 41F | >2.000 | 0.396 | + |
| 8 | L C | 62F | >2.000 | 0.393 | + |
| 9 | L C | 72M | >2.000 | 0.211 | + |
| 10 | L C | 56F | >2.000 | 0.126 | + |
| 11 | H C C | 52M | >2.000 | 0.061 | + |

*  CH = Chronic Hepatitis
*  LC = Liver Cirrhosis
*  HCC = Hepatocellular Carcinoma
** F = Female
** M = Male

Anti-CP9 was found in 13 cases out of 19 cases (68%) of sporadic acute NANB hepatitis and 15 cases out of 18 cases (83%) of post-transfusion NANB hepatitis.

In case of chronic NANB liver diseases, 103 cases out of 133 cases (77%) of patients with chronic hepatitis, 70 cases out of 113 cases (62%) of patients with liver cirrhosis, and 31 cases out of 41 cases (76%) of patients with hepatocellular carcinoma were found positive for anti-CP9.

On the other hand, only one case out of eight cases of acute hepatitis A patients was weakly positive and all of 11 cases of acute hepatitis B patients were negative for this antibody. 7% patients with chronic hepatitis, 12% of patients with liver cirrhosis, and 14% of patients with hepatocellular carcinoma of hepatitis B etiology were positive for this antibody.

All cases of auto-immune diseases, six cases of lupoid hepatitis, and nine cases of primary biliary liver cirrhosis were negative for this antibody.

In comparison of the above data with the data by Chiron Corporation (Science (1989), 244: 362-366) that 70.8% (17 cases out of 24 cases) of post-transfusion acute NANB hepatitis and 56.5% (of cases out of 58 cases) of sporadic NANB hepatitis were positive for the anti-HCV antibody, it was concluded that anti-CP9 could be detected at a much superior and higher rate in acute hepatitis cases.

According to data on Japanese patients (Ortho Diagnostic Systems, Tokyo: Clinical Study), 2.4% (two cases out of 82 cases) of acute hepatitis B patients turned positive for anti-HCV, while, as described above, anti-CP9 under this invention did not cross-react with hepatitis B, thus proving high specificity.

3. Detection of Viral RNA in Samples with Overlapping or Discrepancy Between Ortho's Anti-HCV and Anti-CP9.

Teat results by anti-HCV and anti-CP9 corresponded in 49% of acute NANB hepatitis (at the time of onset of the disease) and 66-67% of chronic hepatitis, liver cirrhosis and hepatocellular carcinoma.

As shown in Table 3, the presence of HCV RNA was confirmed in 10 cases out of 11 cases of chronic NANB hepatitis patients testing negative for anti-HCV but positive for anti-CP9 (absorbance over 2).

From the above results, it was confirmed again that anti-CT9 had higher sensitivity and specificity in diagnosis of chronic NANB hepatitis.

4. Comparison in Detection of Antibody in Acute NANB Hepatitis Samples.

Of three post-transfusion NANB hepatitis and four sporadic NANB hepatitis cases, samples taken at the time of onset of the disease (0 month), three months later and six months later were tested for anti-Cp9 and anti-HCV to compare timing of their sero-conversion. Results are shown in Table 4.

Table 4.  Detection of anti-CP9 and anti-HCV in post-transfusion and sporadic NANB hepatitis patients.

| Infection Source | | Sample No. Age & Sex | | anti-CP9 (upper) and anti-HCV (lower) months after manifestation of disease. Figures are Absorbance. | | |
| --- | --- | --- | --- | --- | --- | --- |
| | | | | 0 Month | 3 Months | 6 Months |
| P O S T - | T R A N S F U S I O N | 1 | 37F | >2.000 | >2.000 | >2.000 |
| | | | | 0.103 | 0.874 | 0.287 |
| | | 2 | 65M | 1.271 | 1.244 | 0.813 |
| | | | | 0.109 | 1.106 | >2.000 |
| | | 3 | 65M | >2.000 | >2.000 | >2.000 |
| | | | | 0.085 | 0.897 | 0.392 |
| | S P O R A D I C | 1 | 33M | 0.758 | >2.000 | >2.000 |
| | | | | 0.103 | 0.476 | 1.078 |
| | | 2 | 43M | >2.000 | >2.000 | >2.000 |
| | | | | 1.531 | >2.000 | >2.000 |
| | | 3 . | 26M | >2.000 | >2.000 | >2.000 |
| | | | | 0.093 | 1.451 | 1.555 |
| | | 4 | 26M | >2.000 | >2.000 | >2.000 |
| | | | | 0.062 | 0.075 | 1.196 |

For all of the post-transfusion NANB hepatitis and sporadic NANB hepatitis cases, anti-CP9 was positive at the time of onset of the disease, while only one case out of seven cases (sporadic hepatitis) was positive for anti-HCV at the time of onset of the disease, five cases turned positive after three months and the remaining one case turned positive as late as six months after onset of the disease. This confirms that the test for anti-CP9 is capable of detecting specific antibody appearing in an early stage of acute NANB hepatitis much faster than anti-HCV.

From the above, it can be said that without relying upon the conventional diagnostic method, anti-CP9 has made it possible to diagnose acute NANB hepatitis.

Experimental Example.

An example of determination of NANB hepatitis virus antibody by peptide having the amino acid sequence of CP-9 (P5-23, 19 mer) is shown below. Determination method is same as that shown in Example 2.

Anti-CP9 and anti-CP10 showed similar frequency (Table 5), however, as for the sample No. 6, it was positive for anti-CP10 while it was negative for anti-CP9, suggesting that anti-CP10 was complementary to anti-CP9.

Table 5.  Detection of anti-CP9, anti-HCV and HCV-RNA in patients with chronic NANB hepatitis positive for anti-CP10.

| Sample No. | Diagnosis* | Age & Sex** | Absorbance (A492) | | | HCV-RNA (PCR) |
|---|---|---|---|---|---|---|
| | | | anti-CP10 | anti-CP9 | anti-HCV | |
| 1 | C H | 46M | >2.000 | >2.000 | 0.031 | + |
| 2 | C H | 44M | >2.000 | >2.000 | 0.076 | + |
| 3 | H C C | 51M | >2.000 | >2.000 | 0.065 | + |
| 4 | C H | 45M | 1.024 | >2.000 | 0.030 | + |
| 5 | L C | 55F | 0.980 | >2.000 | 0.126 | + |
| 6 | L C | 60M | >2.000 | 0.164 | >2.000 | + |
| 7 | L C | 69M | 1.860 | 1.834 | >2.000 | + |
| 8 | L C | 61M | 1.288 | >2.000 | >2.000 | + |
| 9 | H C C | 65M | 1.345 | >2.000 | 1.213 | + |
| 10 | L C | 54M | 1.196 | >2.000 | >2.000 | + |

* CH = Chronic Hepatitis
* LC = Liver Cirrhosis
* HCC = Hepatocellular Carcinoma
** F = Female
** M = Male

Samples Nos. 1 to 5 were negative for anti-HCV (Chiron Corp.) but were positive for anti-CP10 and anti-CP9, and NANB hepatitis viral RNA was detected by PCR in all of them. Thus the detections systems using anti-CP10 is superior to anti-HCV test kit both in sensitivity and specificity.

EP 0 485 209 A1

Experimental Example.

An example of determination of NANB hepatitis viral antibody using peptide having the amino acid sequence of CP-5 (P101-120, 20 mer) is shown below. Determination method is same as that shown in Example 2.

When anti-CP5 and anti-CP9 wore compared, anti-CP9 showed higher frequency than anti-CP5 in a series of samples. However, anti-CP5 was positive for sample nos. 8 to 10 for which anti-CP9 was negative and was considered to be complementary to anti-CP9. Sample Nos. 5 to 7 were negative for anti-HCV but positive for anti-CP5 and anti-CP9 and presence of NANB hepatitis viral RNA was confirmed by PCR. This proved higher specificity and sensitivity of the detection system using CP-5 over anti-HCV detection kit (table 6).

Table 6. Determination of anti-CP9, anti-HCV and HCV-RNA in patients with chronic NANB hepatitis positive for anti-CP5.

| Sample No. | Diagnosis* | Age & Sex** | Absorbance (A492) | | | HCV-RNA (PCR) |
|---|---|---|---|---|---|---|
| | | | anti-CP5 | anti-CP9 | anti-HCV | |
| 1 | C H | 55M | >2.000 | >2.000 | >2.000 | + |
| 2 | C H | 65M | >2.000 | >2.000 | >2.000 | + |
| 3 | C H | 65F | >2.000 | >2.000 | >2.000 | + |
| 4 | L C | 66F | >2.000 | >2.000 | >2.000 | + |
| 5 | C H | 66F | >2.000 | >2.000 | 0.183 | + |
| 6 | C H | 61F | 0.934 | >2.000 | 0.163 | + |
| 7 | L C | 63M | 1.052 | >2.000 | 0.403 | + |
| 8 | L C | 63M | 1.688 | 0.249 | >2.000 | + |
| 9 | C H | 58M | 0.639 | 0.252 | >2.000 | + |
| 10 | L C | 49M | 1.174 | 0.241 | 0.391 | + |

\* CH = Chronic Hepatitis
\* LC = Liver Cirrhosis
\* HCC = Hepatocellular Carcinoma
\*\* F = Female
\*\* M = Male

This invention a makes possible detection of NANB hepatitis virus infection which could not be detected by conventional determination methods, and provide NANB hepatitis detection kits capable of highly specific and sensitive detection at an early phase of infection.

These features allow accurate diagnosis of patients at an early stage of the disease and also help to remove at higher rate NANB hepatitis virus carrier bloods through screening test of donor bloods.

Polypeptides and their antibodies under this invention can be utilized for manufacture of vaccines and immunological pharmaceuticals, and structural gene of NANB hepatitis virus provides indispensable tools for detection of polypeptide antigens and antibodies.

Antigen-antibody complexes can be detected by methods known in this art.

Further variations and modifications of the invention will become apparent to those skilled in the art from the foregoing and are intended to be encompassed by the claims appended hereto.

Japanese Priority Application 304405/90, filed November 8, 1990, is relied on and incorporated by reference. U.S. patent applications serial no. 07/540,604 (filed June 19, 1990), 07/653,090 (filed February 8, 1991), and 07/712,875 (filed June 11, 1991) are incorporated by reference in their entirety.

## Claims

1. Recombinant cDNA of non-A, non-B hepatitis virus, strain HC-J5, comprising the following nucleotide sequence:

```
        A C C C G C C      C C C T A A T A G G

    G G C G A C A C T C     C G C C A T G A A C

    C A C T C C C C T G     T G A G G A A C T A

    C T G T C T T C A C     G C A G A A A G C G

    T C T A G C C A T G     G C G T T A G T A T

    G A G T G T C G T A     C A G C C T C C A G 117

    G C C C C C C C C T     C C C G G G A G A G

    C C A T A G T G G T     C T G C G G A A C C

    G G T G A G T A C A     C C G G A A T T G C

    C G G G A A G A C T     G G G T C C T T T C

    T T G G A T A A A C     C C A C T C T A T G 217

    C C C G G C C A T T     T G G G C G T G C C

    C C C G C A A G A C     T G C T A G C C G A

    G T A G C G T T G G     G T T G C G A A A G

    G C C T T G T G G T     A C T G C C T G A T

    A G G G T G C T T G     C G A G T G C C C C 317

    G G G A G G T C T C     G T A G A C C G T G

    C A C C A T G A G C     A C A A A T C C C A

    A A C C T C A A A G     A A A A A C C A A A

    A G A A A C A C T A     A C C G T C G C C C

    A C A A G A C G T T     A A G T T T C C G G 417

    G C G G C G G C C A     G A T C G T T G G C

    G G A G T A T A C T     T G T T G C C G C G
```

```
CAGGGGCCCC    AGGTTGGGTG
TGCGCGCGAC    AAGGAAGACT
TCGGAGCGGT    CCCAGCCACG 517
TGGGAGGCGC    CAGCCCATCC
CCAAAGATCG    GCGCTCCACT
GGCAAGTCCT    GGGGAAAGCC
AGGATATCCC    TGGCCCCTAT
ATGGGAATGA    GGGGCTCGGC 617
TGGGCAGGAT    GGCTCCTGTC
CCCCCGAGGT    TCCCGTCCCT
CTTGGGGCCC    CAATGACCCC
CGGCATAGGT    CGCGCAATGT
GGGTAAGGTC    ATCGATACCC 717
TAACGTGCGG    CTTTGCCGAC
CTCATGGGGT    ACATCCCCGT
CGTGGGCGCC    CCGCTTGGTG
GCGTCGCCAG    AGCTCTCGCA
CACGGCGTGA    GAGTCCTGGA 817
GGACGGGGTT    AACTATGCAA
CAGGGAACTT    ACCTGGTTGC
TCCTTTTCTA    TTTTCTTGCT
GGCGCTACTG    TCCTGCATCA
CCGTCCCGGT    CTCTGCTGTC 917
CAGGTTAAGA    ACACCAGTAA
CAGCTATATG    GTGACTAACG
ACTGTTCCAA    TGACAGCATT
```

```
A C C T G G C A G C          T C C A G G G C G C

G G T T C T C C A C          G T C C C C G G G T 1017

G C G T C C C G T G          C G A G A A A G T G

G G G A A T A T G T          C A C G G T G C T G

G A T A C C G G T C          T C A C C G A A C G

T G G C T G T G C G          G C A G C C C G G C

G C C C T C A C G C          A G G G T C T G C G 1117

G A C G C A C A T C          G A C A T G G T T G

T G G T G T C C G C          T A C G C T C T G C

T C C G C T C T C T          A C G T G G G G A

C C T C T G C G G T          G G G G T G A T G C

T C G C G G C C C A          G A T G T T C A T C 1217

G T C T C G C C G C          A G C A C C A C T G

G T T T G T G C A G          G A A T G C A A T T

G C T C C A T C T A          C C C T G G T G C C

A T C A C T G G A C          A C C G T A T G G C

A T G G G A C A T G          A T G A T G A A C T 1317

G G T C A C C C A C          G G C C A C C A T G

A T C C T G G C G T          A C G C G A T G C G

C G T C C C C G A G          G T T A T C A T A G

A C A T C A T T A G          C G G G G C T C A C

T G G G G C G T C A          T G T T C G G C C T 1417

A G C C T A C T T C          T C T A T G C A G G

G A G C G T G G G C          G A A G G T C G T T

G T C A T C C T T C          T G C T G G C C G C

T G G A G T G G A T          G C G A A C A C C C
```

34

```
G C A C C G T T G C        G G G T T C T G C T  1517

G C G G C A A C C A        C C A G G G G C T T

C A C C A G C A T G        T T C T C C T C T G

G C T C G A A G C A        G A A C C T T C A G

C T C A T T A A C A        C C A A C G G T A G

C T G G C A C A T C        A A C C G C A C T G  1617

C C C T G A A T T G        C A A T G A C T C C

T T G A A C A C C G        G C T T T A T C G C

G T C C C T G T T C        T A C G T C A A C C

G C T T C A A T T C        G T C A G G A T G T

C C C C A T C G C C        T G T C C G T C T G  1717

C C G C A G C A T C        G A G G C T T T C C

G G A T A G G G T G        G G G C A C C T T G

C A A T A C G A G G        A T A A T G T C A C

C A A T C C A G A A        G A T A T G A G A C

C A T A T T G C T G        G C A C T A C C C A  1817

C C A A A A C C G T        G T G G C A T A G T

C C C C G C G A G G        T C T G T G T G C G

G C C C A G T G T A        C T G T T T C A C C

C C C A G C C C A  1886.
```

2.  Amino acid sequences corresponding to recombinant cDNA of non-A, non-B hepatitis virus, strain HC-J5, comprising the following amino acid sequence:

```
M S T N P K P Q R K        T K R N T N R R P Q

D V K F P G G G Q I        V G G V Y L L P R R
```

```
G  P  R  L  G  V  R  A  T  R        K  T  S  E  R  S  Q  P  R  G

R  R  Q  P  I  P  K  D  R  R        S  T  G  K  S  W  G  K  P  G

Y  P  W  P  L  Y  G  N  E  G        L  G  W  A  G  W  L  L  S  P  100

R  G  S  R  P  S  W  G  P  N        D  P  R  H  R  S  R  N  V  G

K  V  I  D  T  L  T  C  G  F        A  D  L  M  G  Y  I  P  V  V

G  A  P  L  G  G  V  A  R  A        L  A  H  G  V  R  V  L  E  D

G  V  N  Y  A  T  G  N  L  P        G  C  S  F  S  I  F  L  L  A

L  L  S  C  I  T  V  P  V  S        A  V  Q  V  K  N  T  S  N  S  200

Y  M  V  T  N  D  C  S  N  D        S  I  T  W  Q  L  Q  G  A  V

L  H  V  P  G  C  V  P  C  E        K  V  G  N  M  S  R  C  W  I

P  V  S  P  N  V  A  V  R  Q        P  G  A  L  T  Q  G  L  R  T

H  I  D  M  V  V  V  S  A  T        L  C  S  A  L  Y  V  G  D  L

C  G  G  V  M  L  A  A  Q  M        F  I  V  S  P  Q  H  H  W  F  300

V  Q  E  C  N  C  S  I  Y  P        G  A  I  T  G  H  R  M  A  W

D  M  M  M  N  W  S  P  T  A        T  M  I  L  A  Y  A  M  R  V

P  E  V  I  I  D  I  I  S  G        A  H  W  G  V  M  F  G  L  A

Y  F  S  N  Q  G  A  W  A  K        V  V  V  I  L  L  L  A  A  G

V  D  A  N  T  R  T  V  A  G        S  A  A  A  T  T  R  G  F  T  400

S  M  F  S  S  G  S  K  Q  N        L  Q  L  I  N  T  N  G  S  W

H  I  N  R  T  A  L  N  C  N        D  S  L  N  T  G  F  I  A  S

L  P  Y  V  N  R  F  N  S  S        G  C  P  H  R  L  S  V  C  R

S  I  E  A  F  R  I  G  W  G        T  L  Q  Y  E  D  N  V  T  N

P  E  D  M  R  P  Y  C  W  H        Y  P  P  K  P  C  G  I  V  P  500

A  R  S  V  C  G  P  V  Y  C        F  T  P  S  P.
```

3. Recombinant peptide CP-10 of non-A, non-B hepatitis virus, strain HC-J1, comprising the following peptide sequence : PKP QRK TKR NTN RRP QDV K.

4. Recombinant peptide CP-5 of non-A, non-B hepatitis virus, strain HC-J1, comprising the following peptide sequence: RGS RPS WGP TDP RRR SRN LG.

5. The recombinant peptide CP-5 according to claim 4, wherein the 10th, 14th, and 19th amino acids have been replaced by N, H, and V respectively.

6. Recombinant polynucleotide CN-10 of non-A, non-B hepatitis virus, strain HC-J1, comprising the following DNA sequence: CCC AAAC CTC AAA GAAA AAC CAA ACGT AAC ACC AACC GTC GCC CACA GCA CGT CAAG.

7. Recombinant polynucleotide CN-5 of non-A, non-B hepatitis virus, strain HC-J1, comprising the following DNA sequence: CGT GGCTCT CGG CCT AGTT GGG GCC CCAC GGA CCC CCGG CGT AGG TCGC GCA ATT TGGG T.

8. A non-A, non-B hepatitis diagnostic test kit for analyzing samples for the presence of antibodies directed against a non-A, non-B hepatitis antigen, comprising the amino acid sequence according to claim 3 as antigen attached to a solid substrate and labeled anti-human immunoglobulin.

9. A method of detecting antibodies directed against a non-A, non-B hepatitis antigen in a sample, said method comprising:
(a) reacting said sample with the amino acid sequence according to claim 3 to form antigen-antibody complexes; and
(b) detecting said antigen-antibody complexes.

10. A non-A, non-B hepatitis diagnostic test kit for analysing samples for the presence of antibodies directed against a non-A, non-B hepatitis antigen, comprising the amino acid sequence according to claim 4 as antigen attached to a solid substrate and labeled anti-human immunoglobulin.

11. A method of detecting antibodies directed against a non-A, non-B hepatitis antigen in a sample, said method comprising:
(a) reacting said sample with the amino acid sequence according to claim 4 to form antigen-antibody complexes; and
(b) detecting said antigen-antibody complexes.

12. A non-A, non-B hepatitis specific monoclonal or polyclonal antibody reactive with an antigen, said antigen comprising the amino acid sequence according to claim 3.

13. A method of detecting non-A, non-B hepatitis antigen in a sample, said method comprising:
(a) reacting said sample with the non-A, non-B hepatits monoclonal or polyclonal antibody according to claim 12 to form antigen-antibody complexes; and
(b)detecting said antigen-antibody complexes.

14. A non-A, non-B hepatitis specific monoclonal or polyclonal antibody reactive with an antigen, said antigen comprising the amino acid sequence according to claim 4.

15. A method of detecting non-A, non B hepatitis antigen in a sample, said method comprising:
(a) reacting said sample with the non-A, non-B hepatits monoclonal or polyclonal antibody according to claim 14 to form antigen-antibody complexes; and
(b) detecting said antigen-antibody complexes.

Fig. 1 - (a)

Fig. 1 - (b)

EP 0 485 209 A1

Fig. 1 - (c)

Fig. 2

Hydrophilicity vs Amino acid sequence

39

Fig. 3

Shaded area = ALT level, white dot = CP-10,
black dot = anti-HCV

European Patent
Office

# EUROPEAN SEARCH REPORT

Application Number

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 388 232 (CHIRON CORPORATION)<br>* Whole document *<br>--- | 1-15 | C12N15/40<br>C12Q1/70<br>C07K15/00 |
| X | JAPAN JOURNAL EXPERIMENTAL MEDECINE<br>vol. 60, no. 3, 1990, JAPAN<br>pages 167 - 177;<br>H. OKAMOTO ET AL.: 'The 5'-terminal sequence of the hepatitis C virus genome'<br>* Whole article * | 1-7 | G01N33/576 |
| Y | ---<br> | 8-15 | |
| D,Y | EP-A-0 318 216 (CHIRON CORPORATION)<br>* Whole document *<br>--- | 8-15 | |
| X | NUCLEIC ACIDS RESEARCH.<br>vol. 18, no. 15, 1990, LONDON, GB<br>page 4626;<br>K. TAKEUCHI ET AL.: 'Nucleotide sequence of core and envelope genes of the hepatitis C virus genome derived directly from human healthy carriers'<br>* Whole article *<br>--- | 1-7 | |
| P,X | EP-A-0 445 423 (ABBOTT LABORATORIES)<br>* Whole document *<br>--- | 3-4,6-15 | **TECHNICAL FIELDS SEARCHED (Int. Cl.5 )**<br>C07K<br>C12N<br>C12Q |
| P,X | EP-A-0 419 182 (A. OYA ET AL.)<br>* Whole document *<br>--- | 1-15 | |
| P,X | GB-A-2 239 245 (THE WELLCOME FOUNDATION LIMITED)<br>* Whole document *<br>---<br>-/-- | 1-15 | |

The present search report has been drawn up for all claims

| Place of search | Date of completion of the search | Examiner |
|---|---|---|
| BERLIN | 25 FEBRUARY 1992 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)

European Patent
Office

**EUROPEAN SEARCH REPORT**

Application Number

EP 91 31 0297
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | PROCEEDINGS OF THE NATIONAL ACADEMY OF SCIENCES OF USA. vol. 88, no. 8, April 1991, WASHINGTON US pages 3392 – 3396; N. OGATA ET AL.: 'Nucleotide sequence and mutation rate of the H strain of hepatitis C virus' * Whole article * | 1-7 | |

TECHNICAL FIELDS
SEARCHED (Int. Cl.5)

The present search report has been drawn up for all claims

| Place of search | Date of completion of the search | Examiner |
|---|---|---|
| BERLIN | 25 FEBRUARY 1992 | JULIA P. |

EPO FORM 1503 03.82 (P0401)

**CATEGORY OF CITED DOCUMENTS**

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.........................................................................................

& : member of the same patent family, corresponding
   document